(19) Europäisches Patentamt
European Patent Office
Office européen des brevets

(11) Veröffentlichungsnummer: **0 250 362 B1**

(12) **EUROPÄISCHE PATENTSCHRIFT**

(45) Veröffentlichungstag der Patentschrift: **02.01.92**

(51) Int. Cl.[5]: **C07C 221/00**, C09B 1/50

(21) Anmeldenummer: **87810332.4**

(22) Anmeldetag: **10.06.87**

# (54) Verfahren zur Herstellung eines Gemisches von 1-Amino-2-chlor-4-hydroxyanthrachinon und 1-Amino-2-brom-4-hydroxyanthrachinon.

(30) Priorität: **16.06.86 CH 2421/86**

(43) Veröffentlichungstag der Anmeldung:
**23.12.87 Patentblatt 87/52**

(45) Bekanntmachung des Hinweises auf die Patenterteilung:
**02.01.92 Patentblatt 92/01**

(84) Benannte Vertragsstaaten:
**CH DE FR GB IT LI**

(56) Entgegenhaltungen:
**EP-A- 0 166 684**
**DE-A- 1 644 622**
**DE-A- 2 243 197**
**DE-A- 2 428 337**
**DE-A- 2 817 890**

(73) Patentinhaber: **CIBA-GEIGY AG**
**Klybeckstrasse 141**
**CH-4002 Basel(CH)**

(72) Erfinder: **Müller, Rolf**
**Herrenweg 77**
**CH-4147 Aesch(CH)**
Erfinder: **Dill, Bernd, Dr.**
**Brunnenweg 19**
**CH-7888 Rheinfelden 4(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft ein Verfahren zur Herstellung eines Gemisches von 1-Amino-2-chlor-4-hydroxyanthrachinon und 1-Amino-2-brom-4-hydroxyanthrachinon mit einem hohen Anteil an 1-Amino-2-chlor-4-hydroxyanthrachinon.

Bei der Chlorierung von 1-Aminoanthrachinon zu 1-Amino-2,4-dichloranthrachinon entstehen in der Regel Nebenprodukte wie z.B. einfach oder dreifach chlorierte Verbindungen. Diese erschweren die Gewinnung von genügend reiner 1-Amino-2,4-dichlorverbindung und 1-Amino-2-chlor-4-hydroxyanthrachinon erheblich, welches als wichtiges Zwischenprodukt zur Herstellung von Farbstoffen, insbesondere Dispersionsfarbstoffen, benötigt wird. Dadurch müssen Reinigungsoperationen nach der Chlorierung wie z.B. Filtrationen ausgeführt werden, die jedoch zu einem Ausbeuteverlust von ungefähr 15 bis 20 % der Theorie führen.

Aufgabe der vorliegenden Erfindung ist es daher, die Halogenierung von 1-Aminoanthrachinon so zu modifizieren, dass die anschliessende Umsetzung zur entsprechenden 2-Halogen-4-hydroxyanthrachinonverbindung ohne Komplikationen und Reinigungsoperationen in möglichst hoher Ausbeute durchführbar ist.

Erfindungsgemäss wird die Aufgabe dadurch gelöst, dass man 1-Aminoanthrachinon mit Chlor und Brom behandelt und die 1-Amino-2,4-dihalogenanthrachinone in einer zweiten Stufe mit konzentrierter oder rauchender Schwefelsäure umsetzt.

Gegenstand der vorliegenden Erfindung ist daher ein Verfahren zur Herstellung eines Gemisches von 1-Amino-2-chlor-4-hydroxyanthrachinon und 1-Amino-2-brom-4-hydroxyanthrachinon mit einem hohen Anteil an 1-Amino-2-chlor-4-hydroxyanthrachinon, dadurch gekennzeichnet, dass man 1-Aminoanthrachinon in einem inerten organischen Lösungsmittel chloriert bis 60 bis 90 % 1-Amino-2,4-dichloranthrachinon vorliegen, die erhaltene Reaktionsmischung bromiert bis weniger als 2 % Ausgangsprodukt und monohalogeniertes Produkt vorhanden sind, die gebildeten Produkte anschliessend mit konzentrierter oder rauchender Schwefelsäure zum 1-Amino-2-chlor-4-hydroxyanthrachinon und 1-Amino-2-brom-4-hydroxyanthrachinon umsetzt und das erhaltene Gemisch isoliert.

Weitere Gegenstände der vorliegenden Erfindung sind die erfindungsgemäss hergestellten Gemische sowie die Verwendung dieser Gemische zur Herstellung von Farbstoffen, ferner Gemische an sich, die die erfindungsgemässe Zusammensetzung aufweisen und ihre Verwendung zur Herstellung von Farbstoffen.

Gemäss vorliegender Erfindung wird 1-Aminoanthrachinon so lange chloriert bis in der Reaktionsmischung 60 bis 90 % (die Prozentangaben sind im folgenden als Gewichtsprozente zu verstehen) 1-Amino-2,4-dichloranthrachinon vorliegen. Danach wird diese Reaktionsmischung bromiert bis weniger als (zusammengenommen) 2 % Ausgangsverbindung und monohalogeniertes Produkt vorhanden sind.

Nach dieser Halogenierung liegen 90 bis 95 % des eingesetzten 1-Aminoanthrachinons in Form eines Produktgemisches aus 2,4-dihalogeniertem 1-Aminoanthrachinon vor. Diese dihalogenierten Verbindungen setzen sich zu 60 bis 90 % aus 1-Amino-2,4-dichloranthrachinon, und zu 10 bis 40 % aus 1-Amino-2,4-dibrom- und 1-Amino-2-brom-4-chlor-anthrachinon zusammen.

Es hat sich gezeigt, dass bei zu langer Chlorierung (es liegen dann über 90 % 1-Amino-2,4-dichloranthrachinon vor) in grösserem Masse jene Nebenprodukte entstehen, welche die weitere Umsetzung zur gewünschten 4-Hydroxyverbindung und ihre Reinigung sehr aufwendig gestalten und zu Ausbeuteverlusten führen. Eine Weiterchlorierung ist deshalb zu vermeiden.

Die Umsetzung der dihalogenierten Verbindungen mit der starken Säure kann direkt in der nach der Halogenierung vorliegenden Reaktionsmischung durchgeführt werden. Es ist aber auch möglich, dass die dihalogenierten Verbindungen isoliert werden, bevor man sie mit der starken Säure zur Reaktion bringt. Diese Reaktion verläuft in beiden Fällen in 95 bis 98%iger Ausbeute zum entsprechenden 1-Amino-2-halogen-4-hydroxyanthrachinon, wobei in diesem Gemisch der Anteil an 1-Amino-2-chlor-4-hydroxyanthrachinon 60 bis 90 % und an 1-Amino-2-brom-4-hydroxyanthrachinon 10 bis 40 % ausmacht.

Die üblichen Verfahren liefern nach der Chlorierung eine Ausbeute von ca. 85 % mit einem Gehalt von mehr als 95 % 1-Amino-2,4-dichloranthrachinon. Für die Folgestufe wird eine Ausbeute von ca. 87 % mit einem Gehalt von mehr als 95 % 1-Amino-2-chlor-4-hydroxyanthrachinon erreicht. Somit stehen nur etwa 73 % (bezogen auf eingesetztes 1-Aminoanthrachinon) dieser Verbindung mit einem Gehalt von mehr als 95 % zur Weiterverarbeitung zu einem Farbstoff zur Verfügung.

Erfindungsgemäss wird die Ausbeute an Zwischenprodukt aber stark erhöht. Durch die auf die Chlorierung folgende Bromierung erhöht sich die Ausbeute an problemlos weiter umsetzbarem 1-Amino-2,4-dihalogenanthrachinon auf 93 %, welches in der 2. Stufe in 96 %iger Ausbeute umgesetzt werden kann. Damit erhält man eine Gesamtausbeute an 1-Amino-2-chlor-4-hydroxyanthrachinon und 1-Amino-2-brom-4-hydroxyanthrachinon von 89 % mit einem Gehalt von mehr als 95 %, die dann zur Farbstoffherstellung verwendet werden können.

Aehnlich hohe Ausbeuten ergibt zwar auch die alleinige Bromierung zu 1-Amino-2-brom-4-hydroxyanthrachinon. Es können damit aber nur eine sehr geringe Anzahl von Farbstoffen in für Färbezwecke brauchbarer Form hergestellt werden.

Mit dem erfindungsgemässen Verfahren wird also ein Weg gezeigt, wie auf verfahrenstechnisch wesentlich einfachere Weise und in erheblich grösseren Ausbeuten als bisher das gewünschte Zwischenprodukt hergestellt werden kann. Dieses erlaubt die Herstellung einer grossen Anzahl von Farbstoffen, wie z.B. solcher der Formel

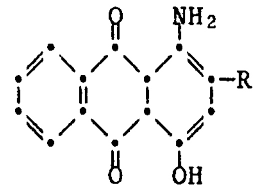 ,

worin R z.B. Wasserstoff, $-OCH_2CH_2OH$, $-(OCH_2CH_2)_{1;2}-OCH_3$,$-(OCH_2CH_2)_{1;2}-OC_6H_5$, $-(OCH_2CH_2)_{1;2}-CN$, $-SCH_2CH_2OH$, $-OC_6H_5$, $-OC_6H_4Cl$, $-OC_6H_4OH$, $-OC_6H_4Br$, $-OC_6H_4SO_2NHCH_2CH_2CH_2OCH_2CH_3$, $-OCH_2CH_2OC_6H_5$, $-OC_6H_4SCH_3$, $-OC_6H_4OCH_3$, $-OC_6H_4C_{10}H_{21}$, $-OC_6H_4OSO_2C_6H_4CH_3$, $-OCH_2CH_2OCONHC_6H_5$, $-OC_6H_4CH_2NHCO_2C_3H_7$,

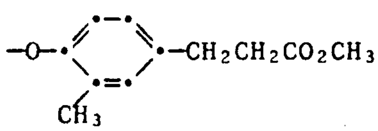 , 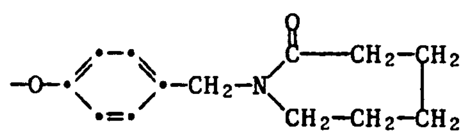 ,

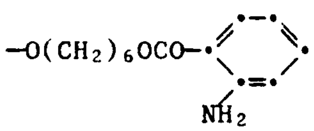 , 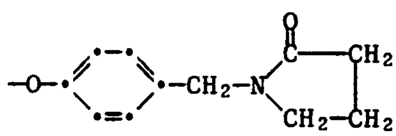 ,

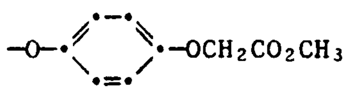 , 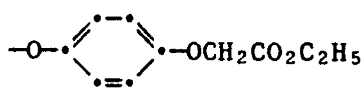 ,

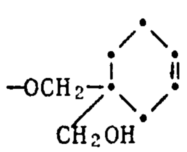 oder 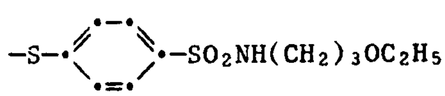

bedeutet.

In einer bevorzugten Ausführungsform der vorliegenden Erfindung werden die nach der Bromierung gebildeten Produkte isoliert und in einem getrennten Reaktionsschritt mit der konzentrierten oder rauchenden Schwefelsäure umgesetzt.

In einer weiteren bevorzugten Ausführungsform wird die Bromierung gleichzeitig mit der Chlorierung durchgeführt. Dabei werden pro Mol 1-Aminoanthrachinon 1,2 bis 2,5 Mol Chlor und 0,05 bis 0,5 Mol Brom eingesetzt.

In einer weiteren bevorzugten Ausführungsform werden die nach der Halogenierung in der Reaktionsmischung enthaltenen 2,4-dihalogenierten Verbindungen mit besagter starker Säure extrahiert und in der dann vorliegenden Phase zu den entsprechenden 2-Halogen-4-hydroxyverbindungen umgesetzt. Die Vorteile dieser Verfahrensweise sind z.B. darin zu sehen, dass die Isolierung und gegebenenfalls Trocknung der dihalogenierten Produkte nach der Halogenierungsstufe entfallen können.

Die Halogenierung des 1-Aminoanthrachinons erfolgt in einem für Flüssigphasenchlorierungen und

-bromierungen üblichen inerten organischen Lösungsmittel, wie z.B. Toluol, Xylol, Mono-, Di- oder Trichlorbenzol, Nitrobenzol, Dimethylformamid, Essigsäure, Trifluoressigsäure oder Mischungen dieser Lösungsmittel, wobei die Reaktionstemperatur vorzugsweise 50 bis 150, insbesondere 100 bis 105 °C beträgt.

Die Chlorierung kann sowohl drucklos als auch unter Ueber- oder Unterdruck ausgeführt werden, wobei man vorzugsweise drucklos arbeitet. Bei der Chlorierung kann flüssiges oder gasförmiges Chlor verwendet werden. Gasförmiges Chlor kann sowohl über als auch unter Niveau in die organische Lösung des 1-Aminoanthrachinons eingeleitet werden.

Brom wird als Lösung in einem der genannten Lösungsmittel oder vorzugsweise in elementarer Form verwendet.

Gegebenenfalls erfolgt die Chlorierung und Bromierung in Gegenwart eines der üblicherweise bei aromatischen Substitutionen verwendeten Katalysatoren wie Jod, Aluminium-, Eisen- oder Antimonchlorid.

Die erfindungsgemässe Halogenierung lässt sich sowohl satzweise, z.B. in einem Rührkessel, oder auch kontinuierlich nach dem bekannten Gegenstromprinzip durchführen, indem 1-Aminoanthrachinon z.B. über Raschigringe, die aus Eisen bestehen können, rieselt und Chlor dazu im Gegenstrom geführt wird.

Nach der Halogenierung erfolgt die Extraktion oder Isolierung der gebildeten Produkte. Für die Isolierung kann man sich üblicher Methoden bedienen, wie z.B. Wasserdampfdestillation oder Eindampfen der Reaktionslösung.

Die so erhaltenen trockenen Produkte werden dann mit der konzentrierten oder rauchenden, vorzugsweise 95 bis 110 %igen Schwefelsäure behandelt. Dazu trägt man die Produkte in die starke Säure ein und erhitzt vorzugsweise auf 80 bis 160, insbesondere 100 bis 140 °C. Es ist vorteilhaft, die Umsetzung in Gegenwart von Borsäure, Bortrioxid oder Aldehyden durchzuführen.

Extrahiert man im erfindungsgemässen Verfahren die in der Halogenierungsstufe gebildeten Produkte mit der starken Säure direkt aus dem organischen Lösungsmittel, so gelten für die weitere Umsetzung dieser Produkte in der erhaltenen Phase die obengenannten Reaktionsbedingungen für die Umsetzung der isolierten, trockenen Produkte und der starken Säure. Dies ist im folgenden Beispiel 5 gezeigt.

Das erfindungsgemäss erhaltene Gemisch von 60 bis 90 % 1-Amino-2-chlor-4-hydroxyanthrachinon und 10 bis 40 % 1-Amino-2-brom-4-hydroxyanthrachinon wird dann auf übliche Weise z.B. durch Ausfällen mit Wasser isoliert, und getrocknet.

Es sei noch erwähnt, dass man die erfindungsgemäss hergestellten Gemische auch durch Abmischen von 60 bis 90 % 1-Amino-2,4-dichloranthrachinon und 10 bis 40 % 1-Amino-2,4-dibrom- und 1-Amino-2-brom-4-chloranthrachinon und Umsetzung dieses Gemisches mit der starken Säure wie oben geschildert sowie direkt durch Abmischen von 60 bis 90 % 1-Amino-2-chlor-4-hydroxy- und 10 bis 40 % 1-Amino-2-brom-4-hydroxyanthrachinon erhalten kann.

Die folgenden Beispiele erläutern die Erfindung, ohne sie darauf zu beschränken. Teile und Prozentangaben beziehen sich auf das Gewicht sofern nicht anders angegeben.

Beispiel 1:

111,5 Teile 1-Aminoanthrachinon werden in
461 Teilen Chlorbenzol auf 100 bis 103 °C erhitzt. Innerhalb 3 Stunden werden bei der gleichen Temperatur
78,1 Teile Chlorgas über Niveau eingeleitet. Nach beendeter Chlorzugabe werden während 1 Stunde tropfenweise
48 Teile Brom zugegeben und eine weitere Stunde bei 100 °C nachgerührt, dann auf 50 °C abgekühlt. Die entstandene kristalline Suspension wird Wasserdampf destilliert, abfiltriert, gewaschen und getrocknet.
Es werden 149,6 Teile eines Gemisches von
74,2 % 1-Amino-2,4-dichloranthrachinon
11,5 % 1-Amino-2-brom-4-chloranthrachinon und
6,6 % 1-Amino-2,4-dibromanthrachinon erhalten.
Die Ausbeute der gewünschten Verbindungen liegt bei 92 % der Theorie. Ein Gemisch von
610 Teilen rauchender Schwefelsäure (108%ig) und
41,5 Teilen Bortrioxyd wird auf 60 °C erwärmt. Sobald eine Lösung vorliegt, werden bei gleicher Temperatur innerhalb einer halben Stunde
149,6 Teile 1-Amino-2,4-dihalogenanthrachinongemisch eingetragen. Bei einem Durchsatz von 10-20 l/h Stickstoff wird 1 Stunde bei 100 °C und 3 Stunden bei 137 bis 140 °C erhitzt. Nach beendeter Umsetzung wird das Reaktionsgemisch auf 70 °C abgekühlt und innerhalb 20 bis 30 Minuten bei 70 bis 90 °C tropfenweise mit
415 Teilen Wasser versetzt und 1 Stunde bei 137 bis 140 °C nachgerührt. Dann wird das ausgefallene Produkt bei 50 °C abfiltriert. Der Filterrückstand wird mit

500 Teilen 60%iger Schwefelsäure und anschliessend mit Wasser neutral gewaschen und getrocknet. Es werden 132,7 Teile 1-Amino-2-chlor/brom-4-hydroxyanthrachinon erhalten.
Dieses Gemisch besteht aus:
78,2 % 1-Amino-2-chlor-4-hydroxyanthrachinon und
11,7 % 1-Amino-2-brom-4-hydroxyanthrachinon und entspricht einer Ausbeute von 95,4 % der Theorie. Die Gesamtausbeute beträgt also 85,6 % der Theorie.

Beispiel 2:

111,5 Teile 1-Aminoanthrachinon werden in
461 Teilen Chlorbenzol auf 100 bis 103 °C erhitzt. Innerhalb 3 Stunden werden bei der gleichen Temperatur
74,6 Teile Chlorgas über Niveau und gleichzeitig
8,0 Teile Brom zugegeben. Es wird eine weitere Stunde bei 100 °C nachgerührt und anschliessend das Reaktionsgemisch Wasserdampf destilliert, abfiltriert, gewaschen und getrocknet.
Es werden 149,3 Teile eines Gemisches von
73,0 % 1-Amino-2,4-dichloranthrachinon
16,1 % 1-Amino-2-brom-4-chloranthrachinon und
1,4 % 1-Amino-2,4-dibromanthrachinon erhalten.
Dies entspricht einer Ausbeute an gewünschten Verbindungen von 90,1 % der Theorie.
148,2 Teile dieses Gemisches werden dann wie in Beispiel 1 angegeben mit konzentrierter Schwefelsäure und Bortrioxyd umgesetzt. Danach isoliert man ein Gemisch von 130,8 Teilen, das 81,6 % 1-Amino-2-chlor-4-hydroxyanthrachinon und 10,5 % 1-Amino-2-brom-4-hydroxyanthrachinon enthält. Dies entspricht einer Ausbeute von 96,8 % der Theorie und einer Gesamtausbeute von 86,6 % der Theorie.

Beispiel 3 (Vergleich):

111,5 Teile 1-Aminoanthrachinon werden in
460 Teilen Chlorbenzol auf 100 bis 103 °C erhitzt. Innerhalb 4 Stunden werden
85,2 Teile Chlorgas über Niveau eingeleitet. Anschliessend destilliert man
50 Teile Chlorbenzol ab, lässt auf Raumtemperatur abkühlen und filtriert das ausgefallene Produkt ab. Der Filterrückstand wird mit
150 Teilen Chlorbenzol gewaschen, anschliessend mit Methanol und Wasser gewaschen und getrocknet. Es werden 121,4 Teile 1-Amino-2,4-dichloranthrachinon erhalten mit einem Gehalt von mehr als 95 %, was einer Ausbeute von 83,1 % der Theorie entspricht.
120,3 Teile dieses Produkts werden wie in Beispiel 1 angegeben mit konzentrierter Schwefelsäure und Bortrioxid umgesetzt. Man erhält so 98,8 Teile 1-Amino-2-chlor-4-hydroxyanthrachinon mit einem Gehalt von mehr als 95 % des gewünschten Produkts. Dies entspricht einer Ausbeute von 87,6 % der Theorie und ergibt somit eine Gesamtausbeute von 68,7 % der Theorie.

Beispiel 4 (Vergleich):

140 Teile 1-Aminoanthrachinon werden in
700 Teile o-Dichlorbenzol eingetragen. Man erhitzt auf 100 bis 103 °C und leitet nach 30 Minuten während 3 Stunden
112,4 Teile Chlorgas unter Niveau ein. Anschliessend werden
130 Teile o-Dichlorbenzol destillativ entfernt. Man lässt auf Raumtemperatur abkühlen und giesst die Reaktionsmischung auf
340 Teile 100%ige Schwefelsäure. Man rührt eine Stunde und trennt dann die o-Dichlorbenzolphase ab. Der Schwefelsäurephase, die 1-Amino-2,4-dichloranthrachinon enthält, wird während 10 Minuten eine Lösung von
68 Teilen Borsäure in 307 Teilen Oleum (25 %) zugesetzt, wobei die Temperatur von 60 bis 65 °C nicht überschritten werden sollte. Danach wird während 1 Stunde auf 100 °C aufgeheizt und 1 Stunde bei dieser Temperatur gerührt. Sodann steigert man die Temperatur innert 1 Stunde auf 137 °C, hält die Reaktionsmischung 3 Stunden lang auf 137 bis 140 °C und kühlt danach auf 110 °C ab. Man lässt
480 Teile Wasser so zulaufen, dass am Ende die Temperatur des Gemisches etwa 130 °C beträgt. Anschliessend lässt man auf 50 °C abkühlen, rührt 2 Stunden bei 50 bis 55 °C, filtriert bei 50 °C und wäscht mit
400 Teilen Schwefelsäure (60 %) nach. Das Nutschgut wird in

1600 Teilen Wasser aufgeschlämmt, bei 90 bis 95°C 2 Stunden lang gerührt und anschliessend bei 50°C filtriert. Nach Waschen mit warmem Wasser wird bei 90 bis 100°C im Vakuum getrocknet. Die Ausbeute an 1-Amino-2-chlor-4-hydroxyanthrachinon à 95 % beträgt 136,7 g (75,7 % der Theorie).

Beispiel 5:

111,5 Teile 1-Aminoanthrachinon werden in
660 Teile o-Dichlorbenzol eingetragen. Man erhitzt auf 100 bis 103°C und leitet während 3 Stunden
74,6 Teile Chlorgas über Niveau ein. Nach beendeter Chlorzugabe werden
8 Teile Brom zugegeben. Man rührt 1 Stunde bei 100°C, lässt auf Raumtemperatur abkühlen und tropft bei 20 bis 30°C während 30 Minuten
548 Teile 100%ige Schwefelsäure hinzu, rührt eine weitere Stunde und trennt dann die o-Dichlorbenzolphase ab. Die Schwefelsäurephase, die ein Gemisch von 1-Amino-2,4-dichloranthrachinon, 1-Amino-2-brom-4-chloranthrachinon und 1-Amino-2,4-dibromanthrachinonenthält, wird mit
72,5 Teilen Oleum (66 %) zu etwa 7%igem Oleum aufkonzentriert und anschliessend mit
52,4 Teilen Borsäure versetzt, wobei die Temperatur 60°C nicht überschreiten sollte. Man heizt danach während 1 Stunde auf 100°C auf und rührt 1 Stunde bei dieser Temperatur. Die Temperatur wird dann während 30 Minuten auf 137 bis 140°C erhöht. Man rührt die Reaktionsmischung 3 Stunden bei dieser Temperatur und kühlt dann auf 110°C ab. Man versetzt so mit
431 Teilen Wasser, dass die Temperatur des Gemisches etwa 130°C und die Schwefelsäurekonzentration etwa 60 % beträgt. Nach einstündigem Rühren bei 130°C kühlt man auf 50°C ab, filtriert bei dieser Temperatur und wäscht mit
300 Teilen Schwefelsäure (60 %) nach. Der Rückstand wird mit Wasser gewaschen und bei 90 bis 100°C im Vakuum getrocknet. Man erhält 125,7 Teile eines Gemisches aus 86,7 % 1-Amino-2-chlor-4-hydroxyanthrachinon und 7,3 % 1-Amino-2-Brom-4-hydroxyanthrachinon, was einer Ausbeute von 85,5 % der Theorie entspricht.

Beispiel 6:

28,2 Teile 1-Amino-2-chlor(brom)-4-hydroxy-anthrachinon - gemäss Beispiel 5 gewonnen - werden in
80 Teile Butan-1,3-diol eingetragen. Man gibt
10,5 Teile Phenol und
11,3 Teile Kaliumcarbonat wasserfrei zu. Danach erwärmt man den Ansatz auf 120°C und rührt während 2 Stunden unter Einleitung von 10 l Stickstoff pro Stunde bei 120°C. Nachher steigert man die Temperatur auf 140-145°C und rührt weitere 4 Stunden unter Einleiten von Stickstoff. Dann kühlt man auf 80-90°C ab und tropft innert ca. 15 Minuten
182 Teile Wasser zu. Dann rührt man bei 80-85°C nach. Nach dem Kühlen auf ca. 25°C wird der pH-Wert durch Zugeben von Schwefelsäure 60 % auf 8,5 gestellt. Das ausgefallene Produkt filtriert man über einen Baumwollfilter und wäscht mit einer Mischung von
28 Teilen Methanol und
39 Teilen Wasser. Anschliessend mit Wasser von 50-60°C waschen, bis der Ablauf farblos und pH-neutral ist. Nach dem Trocknen des Nutschgutes bei 80°C im Vakuum erhält man 28 Teile eines Farbstoffes, der Polyesterfasern in leuchtend rotem Ton färbt. Ausbeute 89,3 % der Theorie.

## Patentansprüche

1. Verfahren zur Herstellung eines Gemisches von 1-Amino-2-chlor-4-hydroxyanthrachinon und 1-Amino-2-brom-4-hydroxyanthrachinon mit einem hohen Anteil an 1-Amino-2-chlor-4-hydroxyanthrachinon, dadurch gekennzeichnet, dass man 1-Aminoanthrachinon in einem inerten organischen Lösungsmittel chloriert bis 60 bis 90 % 1-Amino-2,4-dichloranthrachinon vorliegen, die erhaltene Reaktionsmischung bromiert bis weniger als 2 % Ausgangsprodukt und monohalogeniertes Produkt vorhanden sind, die gebildeten Produkte anschliessend mit konzentrierter oder rauchender Schwefelsäure zum 1-Amino-2-chlor-4-hydroxyanthrachinon und 1-Amino-2-brom-4-hydroxyanthrachinon umsetzt und das erhaltene Gemisch isoliert.

2. Verfahren nach Anspruch 1, dadurch gekennzeichnet, dass man die nach der Bromierung gebildeten Produkte isoliert und sie in einem getrennten Reaktionsschrit mit der konzentrierten oder rauchenden Schwefelsäure umsetzt.

3. Verfahren nach Anspruch 1, dadurch gekennzeichnet, dass man die Chlorierung und Bromierung gleichzeitig ausführt, wobei pro Mol 1-Aminoanthrachinon 1,2 bis 2,5 Mol Chlor und 0,05 bis 0,5 Mol Brom eingesetzt werden.

4. Verfahren nach Anspruch 1, dadurch gekennzeichnet, dass man die gebildeten Produkte mit der Schwefelsäure extrahiert und sie in der erhaltenen Phase direkt zum 1-Amino-2-chlor-4-hydroxyanthrachinon und 1-Amino-2-brom-4-hydroxyanthrachinon umsetzt.

5. Verfahren nach Anspruch 1, dadurch gekennzeichnet, dass die Chlorierung und Bromierung bei einer Temperatur von 50 bis 150 °C erfolgen.

6. Verfahren nach Anspruch 5, dadurch gekennzeichnet, dass die Chlorierung und Bromierung bei einer Temperatur von 100 bis 105 °C erfolgen.

7. Verfahren nach Anspruch 1, dadurch gekennzeichnet, dass die Umsetzung mit der Schwefelsäure bei einer Temperatur von 80 bis 160 °C erfolgt.

8. Verfahren nach Anspruch 7, dadurch gekennzeichnet, dass die Umsetzung mit der Schwefelsäure bei einer Temperatur von 100 bis 140 °C erfolgt.

9. Verfahren nach Anspruch 1, dadurch gekennzeichnet, dass man die Umsetzung mit der Schwefelsäure in Gegenwart von Borsäure, Bortrioxid oder Aldehyden durchführt.

10. Verfahren nach Anspruch 1, dadurch gekennzeichnet, dass als inerte organische Lösungsmittel Toluol, Xylol, Mono-, Di-, Trichlorbenzol, Nitrobenzol, Dimethylformamid, Essigsäure oder Trifluoressigsäure oder Mischungen dieser Lösungsmittel verwendet werden.

11. Verfahren nach Anspruch 10, dadurch gekennzeichnet, dass als inerte organische Lösungsmittel Toluol, Xylol, Mono-, Di-, Trichlorbenzol, Nitrobenzol, Dimethylformamid oder Mischungen dieser Lösungsmittel verwendet werden.

12. Gemisch erhältlich durch das Verfahren gemäß Anspruch 1.

13. Verwendung des Gemisches nach Anspruch 12 zur Herstellung von Farbstoffen.

14. Gemisch, dadurch gekennzeichnet, dass es 60 bis 90 % 1-Amino-2-chlor-4-hydroxyanthrachinon und 10 bis 40 % 1-Amino-2-brom-4-hydroxyanthrachinon enthält.

15. Verwendung des Gemisches nach Anspruch 14 zur Herstellung von Farbstoffen.

## Claims

1. A process for the preparation of a mixture of 1-amino-2-chloro-4-hydroxyanthraquinone and 1-amino-2-bromo-4-hydroxyanthraquinone having a high proportion of 1-amino-2-chloro-4-hydroxyanthraquinone, which comprises chlorinating 1-aminoanthraquinone in an inert organic solvent until 60 to 90% of 1-amino-2,4-dichloroanthraquinone is present, brominating the resultant reaction mixture until it contains less than 2% of starting material and monohalogenated compound, subsequently reacting the products formed with concentrated or fuming sulfuric acid to give 1-amino-2-chloro-4-hydroxyanthraquinone and 1-amino-2-bromo-4-hydroxyanthraquinone, and isolating the mixture obtained.

2. A process according to claim 1, which comprises isolating the products formed after the bromination and reacting them, in a separate reaction step, with the concentrated or fuming sulfuric acid.

3. A process according to claim 1, which comprises carrying out the chlorination and bromination simultaneously, using 1.2 to 2.5 moles of chlorine and 0.05 to 0.5 mole of bromine per mole of 1-aminoanthraquinone.

4. A process according to claim 1, which comprises extracting the products formed with the sulfuric acid

and reacting them, in the resulting phase, direct to 1-amino-2-chloro-4-hydroxyanthraquinone and 1-amino-2-bromo-4-hydroxyanthraquinone.

**5.** A process according to claim 1, wherein the chlorination and bromination are carried out at a temperature from 50 to 150 °C.

**6.** A process according to claim 5, wherein the chlorination and bromination are carried out at a temperature from 100 to 105 °C.

**7.** A process according to claim 1, wherein the reaction with the sulfuric acid is carried out at a temperature from 80 to 160 °C.

**8.** A process according to claim 7, wherein the reaction with the sulfuric acid is carried out at a temperature from 100 to 140 °C.

**9.** A process according to claim 1, wherein the reaction with the sulfuric acid is carried out in the presence of boric acid, boron trioxide or aldehydes.

**10.** A process according to claim 1, wherein the inert organic solvent used is toluene, xylene, mono-, di- or trichlorobenzene, nitrobenzene, dimethylformamide, acetic acid or trifluoroacetic acid, or a mixture of these solvents.

**11.** A process according to claim 10, wherein the inert organic solvent used is toluene, xylene, mono-, di- or trichlorobenzene, nitrobenzene, dimethylformamide, or a mixture of these solvents.

**12.** A mixture obtainable by a process according to claim 1.

**13.** The use of a mixture as claimed in claim 12 for the synthesis of dyes.

**14.** A mixture which contains 60 to 90% of 1-amino-2-chloro-4-hydroxyanthraquinone and 10 to 40% of 1-amino-2-bromo-4-hydroxyanthraquinone.

**15.** The use of a mixture according to claim 14 for the synthesis of dyes.

**Revendications**

**1.** Procédé pour préparer un mélange de 1-amino-2-chloro-4-hydroxyanthraquinone et de 1-amino-2-bromo-4-hydroxyanthraquinone à proportion élevée de 1-amino-2-chloro-4-hydroxyanthraquinone, caractérisé en ce que l'on chlore la 1-aminoanthraquinone dans un solvant organique inerte, jusqu'à obtention de 60 à 90% de 1-amino-2,4-dichloroanthraquinone, on brome le mélange réactionnel obtenu, jusqu'à ce que soient présents moins de 2% du produit de départ et de produit monohalogéné, on fait ensuite réagir les produits formés avec l'acide sulfurique concentré ou fumant, pour obtenir la 1-amino-2-chloro-4-hydroxyanthraquinone et la 1-amino-2-bromo-4-hydroxyanthraquinone, et on isole le mélange obtenu.

**2.** Procédé selon la revendication 1, caractérisé en ce que l'on isole les produits formés après la bromation et on les fait réagir avec l'acide sulfurique concentré ou fumant, dans une étape réactionnelle séparée.

**3.** Procédé selon la revendication 1, caractérisé en ce que l'on effectue simultanément la chloration et la bromation, en employant 1,2 à 2,5 moles de chlore et 0,05 à 0,5 mole de brome, par mole de 1-aminoanthraquinone.

**4.** Procédé selon la revendication 1, caractérisé en ce que l'on extrait les produits formés avec l'acide sulfurique et on les convertit directement, dans la phase obtenue, en 1-amino-2-chloro-4-hydroxyanthraquinone et 1-amino-2-bromo-4-hydroxyanthraquinone.

**5.** Procédé selon la revendication 1, caractérisé en ce que la chloration et la bromation sont réalisées à

une température de 50 à 150° C.

6. Procédé selon la revendication 5, caractérisé en ce que la chloration et la bromation sont réalisées à une température de 100 à 105° C.

7. Procédé selon la revendication 1, caractérisé en ce que la réaction avec l'acide sulfurique est réalisée à une température de 80 à 160° C.

8. Procédé selon la revendication 7, caractérisé en ce que la reaction avec l'acide sulfurique est réalisée à une température de 100 à 140° C.

9. Procédé selon la revendication 1, caractérisé en ce que l'on effectue la réaction avec l'acide sulfurique en présence d'acide borique, de trioxyde de bore ou d'aldéhydes.

10. Procédé selon la revendication 1, caractérisé en ce que l'on utilise, comme solvant organique inerte, le toluène, le xylène, le mono-, le di-, le trichlorobenzène, le nitrobenzène, le diméthylformamide, l'acide acétique ou l'acide trifluoroacétique ou des mélanges de ces solvants.

11. Procédé selon la revendication 10, caractérisé en ce que l'on utilise, comme solvant organique inerte, le toluène, le xylène, le mono-, le di-, le trichlorobenzène, le nitrobenzène, le diméthylformamide ou des mélanges de ces solvants.

12. Mélange qui peut être obtenu au moyen du procédé selon la revendication 1.

13. Utilisation du mélange selon la revendication 12 pour préparer des colorants.

14. Mélange, caractérisé en ce qu'il contient 60 à 90% de 1-amino-2-chloro-4-hydroxyanthraquinone et 10 à 40% de 1-amino-2-bromo-4-hydroxyanthraquinone.

15. Utilisation du mélange selon la revendication 14 pour préparer des colorants.